# EUROPEAN PATENT SPECIFICATION

(11) **EP 3 915 083 B1**
(45) Date of publication and mention of the grant of the patent: **02.12.2026**
(21) Application number: 20744309.4
(22) Date of filing: 23.01.2020
(51) Int. Cl.: G06Q 50/10, G05B 23/02, G01M 5/00, G06N 20/00

(54) **ANOMALY DETECTION FOR PREDICTIVE MAINTENANCE AND DERIVING OUTCOMES AND WORKFLOWS BASED ON DATA QUALITY**
ANOMALIEDETEKTION FÜR DIE VORAUSSCHAUENDE WARTUNG UND ABLEITUNG VON ERGEBNISSEN UND ARBEITSABLÄUFEN BASIEREND AUF DER DATENQUALITÄT
DÉTECTION D'ANOMALIE POUR MAINTENANCE PRÉDICTIVE ET DÉDUCTION DE RÉSULTATS ET DE FLUX DE TRAVAUX SUR LA BASE DE LA QUALITÉ DE DONNÉES

(30) Priority: 25.01.2019 US 201962797076 P
(43) Date of publication of application: 01.12.2021
(73) Proprietor: Bently Nevada, LLC, Houston, TX 77079 (US)
(72) Inventor: GATTU, Jagadish, Lewistown, Pennsylvania 17044 (US); OHAD, Nimrod, Lewistown, Pennsylvania 17044 (US)
(74) Representative: Novagraaf Group
(86) International application number: PCT/US2020/014713
(87) International publication number: WO 2020/154461

(56) References cited:
- JP-B2- 3 468 946
- US-A1- 2007 010 900
- US-A1- 2007 010 900
- US-A1- 2014 214 325
- US-A1- 2016 305 848
- US-A1- 2018 191 867
- US-A1- 2019 003 929
- US-B1- 6 862 540

## Description

### BACKGROUND

Industrial equipment can be complex and can be prone to different types of complex modes of failure. The equipment can include a multitude of sensors that can be used to monitor operation of the equipment. One method of utilizing sensor data includes developing rule-based detection schemes that can be used to monitor performance of the equipment. Based on the rules implemented within the detection schemes, the sensors, or a controller monitoring the sensors, can determine if the equipment is operating within acceptable parameters.

US 2016/305848 A1 discloses autonomous polynomial interpolation of time series data. US 2007/010900 A1 discloses diagnosing operation of an industrial system.

### SUMMARY

The present invention is set forth and characterized in the independent claims, while the dependent claims describe other characteristics of the invention. Methods for sensor health monitoring are provided. The methods can perform sensor health monitoring by verifying data quality and suppressing alerts via machine learning techniques in order to identify whether two anomalies generated by an asset monitoring system are related. Related apparatus, systems, techniques and articles are also described.

### DESCRIPTION OF DRAWINGS

FIG. 1 is a process flow diagram illustrating an example implementation of a process for outcome derivation; and
FIG. 2 is a system diagram illustrating a system for sensor health monitoring.

Like reference symbols in the various drawings indicate like elements.

### DETAILED DESCRIPTION

Oil and gas industrial environments can utilize asset monitoring and diagnosis systems to monitor the operating conditions of one or more, and in some cases, thousands of assets. In asset monitoring and diagnosis systems including sensors, detection of anomalies in sensor measurements can be desirable. Monitoring asset operation can include efficient management of alerts generated in response to anomaly detection. Overall system performance can be determined by the health status of sensors. Sensor and/or instrument health can be relied on by predictive maintenance analyses and/or analytic solutions to diagnose asset health, in some cases, before problems can arise.

Predictive diagnostics to diagnose the health of an asset can, however, include a chance that the asset is misdiagnosed. Further, predictive maintenance analyses and/or analytic solutions not accounting for sensor health can incorrectly misdiagnose assets that are otherwise operating properly. In some cases, duplicate diagnoses can be made, which each can trigger an alert. In either case, an alert can be generated notifying users of the asset monitoring and diagnosis systems of a potentially anomalous asset, but uncertainty can be included in the predictive maintenance analysis diagnosis generating the alert of a potential future failure. Furthermore, duplicate alerts can exhaust user bandwidth and can render an asset monitoring system unusable.

It can be desirable to monitor sensor health to prevent asset health misdiagnosis. And it can be desirable to suppress duplicate alerts resulting from related diagnoses. Some aspects of the current subject matter can facilitate sensor health monitoring by verifying data quality and/or duplicate alert suppression by utilizing machine learning to identify whether anomalies generated by an asset monitoring system are related, for example, two anomalies.

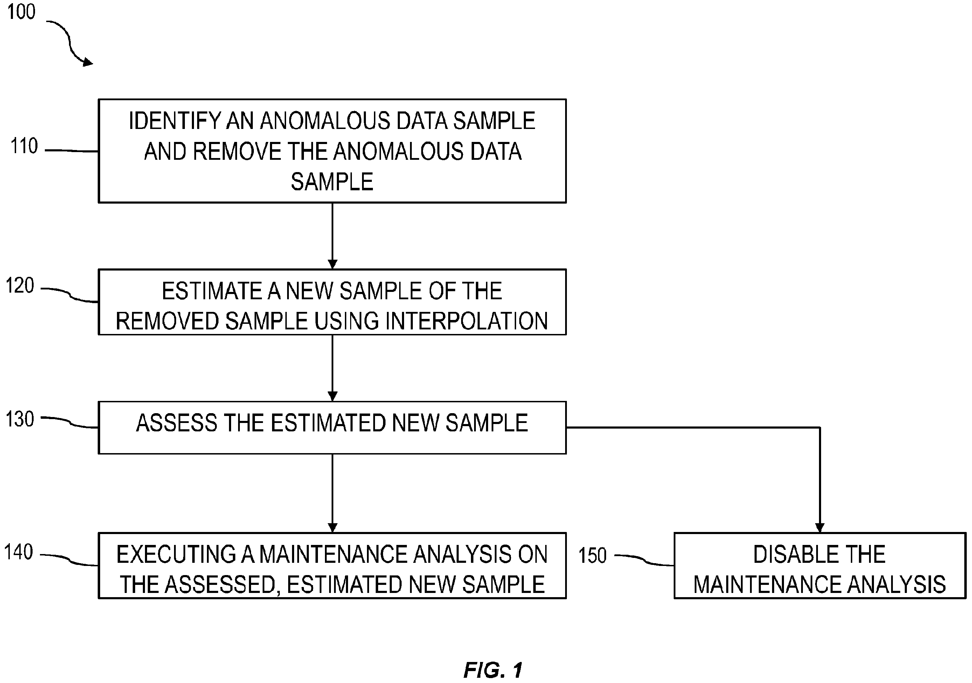

FIG. 1 is a process flow diagram illustrating an example process 100 for outcome derivation for predictive analytics. Utilizing data quality rules can facilitate sensor health monitoring by verifying data quality and/or generating an alert when data quality can be determined to be bad. By verifying data quality and duplicate alert suppression by utilizing machine learning, identification of whether two or more anomalies generated by an asset monitoring system are related is achieved.

At 110, anomalous data samples are identified and removed. The data samples are processed for anomalous data samples in a data quality engine. The data quality engine utilizes data quality or data validation rules to identify anomalous data samples. When a data quality rule is violated (e.g., the data includes bad data quality and thus causes a data quality rule to be broken), a generated alert can identify the start and end of the data quality issue. The generated alert can identify individual data points which have violated the data quality rule. For example, a data validation rule can verify whether the datatype of a signal or measurement received from the sensor matches the datatype of a pre-determined data tag, the signal or measurement received from the sensor contains NULL and/or empty values, and/or the signal or measurement received from the sensor is missing for more than three samples.

A mean variance rule verifies the rate of change of the signal and whether there is a change in the mean value and variance for the rate of change. When the change in mean and variance is considerable, exceeds a threshold, and/or is outside of a predetermined range, the mean variance rule can be violated. For example, an out range rule can verify whether the signal values are within valid operating range. For example, a flat line rule can receive a flat line number and identify how many repeating signals can occur before a signal can be considered flat lined. The flat line rule can receive a flat line precision range and values within the flat line precision range can be considered as repeating signal values. Data samples violating data quality rules are identified as anomalous or problematic, and the identified data samples are removed. In some implementations, metadata identifying the problem associated with anomalous data can be stored along with the time series data in a way that can maintain the size of the time series data (e.g., without significantly increasing the space required to store the time series data).

At 120, removed data samples are estimated using missing data estimation techniques. When a data sample is removed, it can be desirable to estimate the missing data to continue estimating asset operation. Estimating missing data samples can allow asset monitoring even when data can be sparse and/or intermittently lost. Missing data can be estimated using missing data estimation techniques, such as interpolation and/or extrapolation. Interpolation can construct new data samples within the range of a discrete set of existing data. Extrapolation can construct new data samples outside the range of the discrete set of existing data. Data estimation is performed based on gradient boosting.

At 130, estimation of removed data is assessed. According to the inevntion, the estimated data is assessed by partitioning a known dataset into training and/or validation sets and evaluating the fit of the estimated data to the known dataset using f-test. The fit of the estimated data can prevent exhausting user bandwidth understanding the root cause of an alert when the problem can be in the data.

When estimated data can be assessed as a good fit, for example when an estimate quality metric exceeds a pre-determined threshold value or an error metric is below a pre-determined threshold value, at 140, a maintenance analysis on the data samples including the estimated data is executed. If an out of range and/or not an error (NaN) test failure is observed, rules down the line that use these tags generate an alert for data quality violations, but use the estimated data to execute the maintenance rules that are dependent on these tags. For example, threshold rules, anomaly detection, and/or the like can rely on the tags. By performing analytics using the estimated data values, the analytic performance can be improved. Further, identifying the data quality and estimating the missing data can help in detecting specific failure modes for the asset because detecting specific failure modes can require the ability to assess trends in individual sensor data.

When estimated data can be assessed as bad data, at 150, a maintenance analysis on the data samples can be suspended. If an out of range and/or NAN test failure is observed, rules down the line can generate an alert for the data quality violations, and the maintenance rules that can depend on these tags can be suspended. For example, threshold rules, anomaly detection, and/or the like can rely on the tags. By suspending maintenance analytics for poorly estimated data values, scenarios in which the analytic might produce a poor result can be avoided.

In some implementations, when a threshold rule is violated continuously, detecting that the same rule has been violated multiple times, and that multiple alerts should not be continuously generated can be performed. But in the case of predictive maintenance analyses based on machine learning models, the violation can be generated by patterns in several signals. In such a case, there can be no precise rule to detect that two distinct patterns, each generating a separate alert, can correspond to the same problem. Machine learning techniques can be utilized to determine when two diagnoses, based on two separate patterns, are the same diagnosis. Based on this determination, new alert generation can be suppressed so that the user is alerted once, rather than multiple, in some cases hundreds of times, for a specific problem.

In some implementations, generating an alert can combine fuzzy logic and machine learning to identify whether two anomalies generated by an anomaly detection model are the same. The signals contributing to the first anomaly can be analyzed and the pattern of the contributing signals can be compared to the pattern of contributing signals in the second anomaly. If the patterns are a match (using the combination of fuzzy logic and machine learning), then the second anomaly cannot generate a new alert. Instead, the second anomaly information can be added to the alert corresponding to the first anomaly. Thus, one alert can be generated for two separate anomalies identified at two distinct timestamps.

In some implementations, when using predictive diagnostics to diagnose the health of an asset, the nature of the predictive diagnosis can include a chance that the health of the asset is misdiagnosed. As a result, an engineer can be required to assess the validity of the diagnosis. In order to assess the validity, the engineer can look at the overall health of the asset. However, this information can be spread amongst several different systems, such as Computerized Maintenance Management Software (CMMS) and Enterprise Asset Management (EAM), vibration monitoring systems, lube oil analysis, calibration information, and/or the like. This can create a cumbersome, time consuming process to assess a diagnosed problem. It can be desirable for this contextual information, needed for problem assessment, to be presented in one place, for example, in a single pane of glass. But, to reduce user effort and decrease time required to address the diagnosed problem, the contextual information may need to be presented in the context of the current problem.

In some implementations, a layered approach can present the relevant asset health information in a single analysis pane. This approach can enable a user to see maintenance analysis information, varying past alerts, and/or previous failures in a single screen. For example, data quality alerts can be highlighted on a single time series tag. A maintenance analysis record can be displayed on a separate axis with a common time stamp. And, if there is a calibration record, it can be shown as corresponding to a single tag. But, anomalies and failure modes can be displayed as bands within the time series tags, and can highlight only the contributing tags. By filtering the tags to display only the contributing tags, the scope of information displayed to the user can be reduced. And, the maintenance analysis records, which can apply to the entire asset, can be displayed in a separate asset within the same time window giving an overall context to the user.

In some implementations where predictive maintenance analyses can generate an alert about a potential future failure of an oil and gas industrial asset, uncertainty can be included in the diagnosis. Accurate maintenance analysis records and data can be desirable because, for example, shutting down a compressor can be a multi-million dollar decision. As a result, it can be desirable for the uncertainty in a diagnosis to be minimized. But confidence in the existence of a problem can take months to develop. Even though relevant information exists, analysis at scale can be cumbersome. For example, there can be thousands of maintenance records, root cause analysis reports, manuals, and/or the like. It can be desirable to use this data to help an engineer find relevant information that can increase the rate of investigation. It can be cumbersome to assess the validity of a predictive maintenance analysis diagnosis. And searching through billions of structured and unstructured time series data points can be cumbersome. But it can be desirable to utilize this data to find relevant information that can speed up investigations.

In some implementations, natural language based search can be utilized for knowledge management of unstructured records and/or manuals. This can make search easy for a user to find relevant information when searching millions of data records. Machine learning can be utilized to identify past tests or thresholds which have failed and have been determined to match a current problem. Then machine learning can be utilized to identify the matching past problems, including any tests or thresholds which have failed and recommend the identified past problems as the top recommendations for a current problem. Fixes to the past problems corresponding to the current problem can be utilized to efficiently manage alerts. Machine learning can be used to process past time series signals, which can be searched to identify patterns that can be determined to match the current tests or thresholds which have failed. Maintenance analysis actions from the past can be correlated with the current data and can provide a clear idea of past failures that occurred when similar problems were identified.

In some implementations, predictive maintenance analysis solutions can use physics or statistics based models and/or rules to predict the health of an asset. Updating models due to process changes and/or diagnosis false positives can include a significant cost and can be cumbersome to the user. It can be desirable to maintain the models based on process conditions and/or when a diagnosis is incorrect.

In some implementations, unsupervised learning can update predictive models as process conditions change. Smart recommendations can learn whether a diagnosis was correct or incorrect. This information can be used to update the model. For example, the next time a problem is diagnosed, the diagnosis can be more accurate. For process related updates, data can be continuously fed into the model. Whenever the data cannot fit the confidence bands of the model, the model can trigger recalibration. This can result in automatically updating the model. When the user analyzes a problem and specifies that the diagnosis is a false positive, this information can be forwarded to the model. Similarly, to fix the issue, the recommendation due to a synchronization with the work order can be considered to be a true diagnosis. The recommendation can send this information back to the machine learning model and can use this information for reinforced learning of the model. As a result, the model can automatically update.

In some implementations, when a diagnosis is made, operation of the machine undergoing analysis can be modified. For example, when a turbine is diagnosed with anomalous vibrations, the rotation speed of the turbine can be modified in order to correct or address the anomalous behavior. For example, operation of the turbine may be terminated in order to perform maintenance. Other modifications are possible.

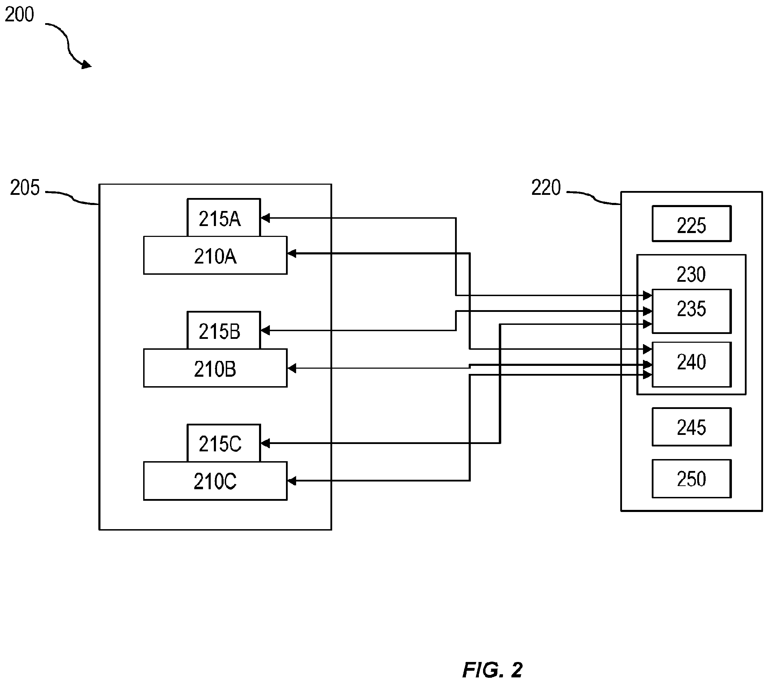

FIG. 2 is a diagram illustrating a system 200 for sensor health monitoring. The system 200 includes an industrial environment 205, such as an oil and gas industrial environment. The industrial environment 205 includes a plurality of industrial assets, shown as industrial asset 210A, 210B, and 210C, which can be collectively referred to as industrial assets 210. The industrial assets can include a variety of equipment or machinery used in a particular industrial domain. For example, the industrial assets 210 can include compressors, pumps, pump motors, heat exchangers, turbines, turbomachinery, or the like. The industrial environment 205 also includes sensors coupled to the plurality of industrial assets 210. The sensors, shown as sensors 215A, 215B, and 215C can be collectively referred to as sensors 215. The sensors 215 can include sensors configured to generate data signals or measurements associated with a vibration, a rotation, an acceleration, an emission, or the like of the industrial assets 210.

As shown in FIG. 2, the system 200 also includes a computing device 220. The computing device 220 can be communicatively coupled to the industrial assets 210 and to the sensors 215. In some embodiments, any of the computing devices 220, the industrial assets 210, and/or the sensors 215 can be coupled via a wired communication means. In some embodiments, the computing device 220 can be coupled to any of the computing devices 220, the industrial assets 210, and/or the sensors 215 via a wireless communication means. In some embodiments, the computing device 220 can be coupled to any of the computing devices 220, the industrial assets 210, and/or the sensors 215 via a network, such as a virtual private network configured to share data within the industrial environment 205.

The computing device 220 includes a data processor 225, a predictive analytic system 230, a memory 245, and a display 250. The predictive analytic system 230 can include computer-readable instructions, rules, and predictive models which when executed by the data processor 225 monitor sensor health by performing the process 100 described in relation to FIG. 1. The predictive analytic system 230 includes a data quality engine 235 and a controller 240. The data quality engine 235 is coupled to the sensors 215 and can receive measurement data from the sensors for use in monitoring the operation and health of the sensors 215 and the assets 210. The data quality engine 235 can include one or more rules used to evaluate and validate the quality of the measurement signals or data received from the sensors 215.

The predictive analytic system 230 also includes a controller 240. The controller 240 is coupled to each of the industrial assets 210 and can be configured to control an operation of the industrial asset 210 based on the maintenance analysis executed by the data quality engine 235 in operation 140 of FIG. 1. The controller 240 can be configured to modify operations such as powering on or powering off the industrial asset 210, adjusting a rate of speed of the industrial asset 210, modifying a frequency of operation of the industrial asset 210, or the like.

The computing device 220 also includes a memory 245. The memory 245 can include a database or other similar data structure which can be used to store computer-readable instructions, data quality or data validation rules, predictive models, as well as sensor data received from the sensors 215 and configuration data associated with controlling the operation of the industrial asset 210 using the controller 240.

The computing device 220 also includes a display 250. The display 250 can include a graphical user interface (not shown). The display 250 can provide the results of the maintenance analysis, any alerts generated by the predictive analytic system 230, and operational data associated with the operation of the industrial asset 210 and/or the sensor 215 to a user or operator of the predictive analytic system 230.

The subject matter described herein can provide many technical advantages. For example, it can facilitate sensor health monitoring by verifying data quality and duplicate alert suppression by utilizing machine learning to identify whether two anomalies generated by an asset monitoring system are related

One or more aspects or features of the subject matter described herein can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system may include clients and servers. A client and server are generally remote from each other and typically interact through a communication network. The relationship of client and server arises by virtue of computer programs running on the respective computers and having a client-server relationship to each other.

These computer programs, which can also be referred to as programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including acoustic, speech, or tactile input. Other possible input devices include touch screens or other touch-sensitive devices such as single or multi-point resistive or capacitive trackpads, voice recognition hardware and software, optical scanners, optical pointers, digital image capture devices and associated interpretation software, and the like.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." In addition, use of the term "based on," above and in the claims is intended to mean, "based at least in part on," such that an unrecited feature or element is also permissible.

The subject matter described herein can be embodied in systems, apparatus, methods, and/or articles depending on the desired configuration. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the implementations described above can be directed to various combinations and subcombinations of the disclosed features and/or combinations and subcombinations of several further features disclosed above. In addition, the logic flows depicted in the accompanying figures and/or described herein do not necessarily require the particular order shown, or sequential order, to achieve desirable results. Other implementations may be within the scope of the following claims.

## Claims

1. A method (100) comprising:
receiving data characterizing measurement data values acquired by a sensor (215) coupled to an industrial asset (210);
**characterized by** identifying (110) an anomalous data sample within the received data, wherein identifying the anomalous data sample includes applying a data validation rule to the received data, the data validation rule configured to verify the quality of the received data and to generate an alert in response to the anomalous data sample failing the data validation rule, wherein the data validation rule verifies a datatype of the measurement data values and a rate of change of the measurement data values, a mean variance rule verifying the rate of change of the measurement data values and whether there is a change in the mean value and variance for the rate of change;
removing the anomalous data sample,
estimating (120), using interpolation, a new sample of the removed data sample, wherein estimating a new sample of the removed data sample is performed based on gradient boosting;
assessing (130) the estimated new sample, wherein assessing the estimated new sample includes evaluating a fit of the estimated new sample with respect to a dataset of known measurement data values, the fit determined based on applying an f-test and/or one or more pre-determined thresholds, wherein responsive to determining the fit of the estimated new sample fails the tests or is not within the one or more pre-determined thresholds, generating an alert including at least one tag identifying the failing test or failing threshold;
executing (140) a maintenance analysis on the assessed, estimated new sample; and
providing a result of the maintenance analysis, wherein the maintenance analysis is executed on the assessed, estimated new sample based on the at least one tag, wherein providing the result of the maintenance analysis includes providing the alert and the at least one tag in a display (250).

2. The method of claim 1, wherein the sensor (215) is affixed to an asset (210) in an oil and gas industrial environment and the data further characterizes a state of health of the asset (210).

3. The method of claim 2, wherein the sensor (215) is included in a sensor health monitoring system (200) associated with the oil and gas industrial environment and the data further characterizes a state of health of the sensor (215).

4. The method of claim 1, wherein the data validation rule further verifies at least one of an empty data value within the measurement data values, a presence of repeating measurement data values, and an absence of three or more measurement data values.

5. The method of claim 1, wherein the generation of alerts is suppressed based on evaluating the alert and the fit using one or more machine learning models trained in a machine learning process utilizing historical measurement data values associated with the industrial asset (210) as training data for the one or more machine learning models.

6. The method of claim 5, wherein the one or more machine learning models are further configured to generate a combined alert responsive to determining the fit of two or more estimated new samples fail one or more tests or thresholds identified as failing previously, wherein the two or more estimated new samples failing the one or more similar tests or thresholds include identical failure patterns.

7. The method of claim 5, wherein the one or more machine learning models can be recalibrated and updated based on the fit of two or more estimated new samples failing one or more tests or thresholds, wherein the training data can include false positive results of the maintenance analysis.

8. The method of claim 1, further comprising, modifying an operation of the industrial asset (210) based on executing the maintenance analysis on the assessed, estimated new sample.

9. A system comprising:
a data processor (225), a display (250), a controller (240), and a memory (245) storing non-transitory, computer-readable instructions, which when executed cause the data processor (225) to perform operations including the method of any one of the preceding claims.

10. A non-transitory computer readable storage medium containing program instructions, which when executed by at least one data processor causes the at least one data processor to perform operations comprising the method of any one of claims 1 to 8.

## Patentansprüche

1. Verfahren (100), umfassend:
Empfangen von Daten, die Messdatenwerte, die durch einen Sensor (215), gekoppelt mit einer Industrieanlage (210), erfasst werden, charakterisieren;
**gekennzeichnet durch** Identifizieren (110) einer anomalen Datenstichprobe innerhalb der empfangenen Daten, wobei das Identifizieren der anomalen Datenstichprobe ein Anwenden einer Datenvalidierungsregel auf die empfangenen Daten einschließt, wobei die Datenvalidierungsregel konfiguriert ist, um die Qualität der empfangenen Daten zu verifizieren und um eine Warnung als Reaktion darauf zu erzeugen, dass die anomale Datenstichprobe die Datenvalidierungsregel nicht erfüllt, wobei die Datenvalidierungsregel einen Datentyp der Messdatenwerte und eine Änderungsrate der Messdatenwerte verifiziert, wobei eine Mittelvarianzregel die Änderungsrate der Messdatenwerte verifiziert und ob es eine Änderung in dem Mittelwert und der Varianz für die Änderungsrate gibt;
Entfernen der anomalen Datenstichprobe,
Schätzen (120), unter Verwendung von Interpolation, einer neuen Stichprobe der entfernten Datenstichprobe, wobei das Schätzen einer neuen Stichprobe der entfernten Datenstichprobe basierend auf Gradientenverstärkung durchgeführt wird;
Bewerten (130) der geschätzten neuen Stichprobe, wobei das Bewerten der geschätzten neuen Stichprobe ein Auswerten einer Passung der geschätzten neuen Stichprobe hinsichtlich eines Datensatzes bekannter Messdatenwerte einschließt, wobei die Passung basierend auf dem Anwenden eines F-Tests und/oder einer oder mehrerer zuvor bestimmter Schwellen bestimmt wird, wobei als Reaktion auf das Bestimmen, dass die Passung der geschätzten neuen Stichprobe die Tests nicht erfüllt oder nicht innerhalb der einen oder der mehreren zuvor bestimmten Schwellen liegt, Erzeugen eines Alarms, einschließlich mindestens eines Tags, das den nicht erfüllten Test oder die nicht erfüllte Schwelle identifiziert;
Ausführen (140) einer Instandhaltungsanalyse an der bewerteten, geschätzten neuen Stichprobe; und
Bereitstellen eines Ergebnisses der Instandhaltungsanalyse, wobei die Instandhaltungsanalyse an der bewerteten, geschätzten neuen Stichprobe basierend auf dem mindestens einen Tag ausgeführt wird, wobei das Bereitstellen des Ergebnisses der Instandhaltungsanalyse das Bereitstellen der Warnung und des mindestens einen Tags in einer Anzeige (250) einschließt.

2. Verfahren nach Anspruch 1, wobei der Sensor (215) an einer Anlage (210) in einer Öl- und Gasindustrieumgebung befestigt ist und die Daten ferner einen Erhaltungszustand der Anlage (210) charakterisieren.

3. Verfahren nach Anspruch 2, wobei der Sensor (215) in einem Sensor-Erhaltungsüberwachungssystem (200), das mit der Öl- und Gasindustrieumgebung verknüpft ist, eingeschlossen ist und die Daten ferner einen Erhaltungszustand des Sensors (215) charakterisieren.

4. Verfahren nach Anspruch 1, wobei die Datenvalidierungsregel ferner mindestens eines von einem leeren Datenwert innerhalb der Messdatenwerte, einem Vorhandensein von sich wiederholenden Messdatenwerten und einem Fehlen von drei oder mehr Messdatenwerten verifiziert.

5. Verfahren nach Anspruch 1, wobei die Erzeugung von Alarmen basierend auf dem Auswerten des Alarms und der Passung unter Verwendung eines oder mehrerer Modelle für maschinelles Lernen unterdrückt wird, die in einem Prozess für maschinelles Lernen, der historische Messdatenwerte, die mit der Industrieanlage (210) verknüpft sind, als Trainingsdaten für das eine oder die mehreren Modelle für maschinelles Lernen nutzt, trainiert sind.

6. Verfahren nach Anspruch 5, wobei das eine oder die mehreren Modelle für maschinelles Lernen ferner konfiguriert sind, um, als Reaktion auf das Bestimmen, dass die Passung von zwei oder mehr geschätzten neuen Stichproben einen oder mehrere Tests oder Schwellen, die zuvor als nicht erfüllt identifiziert wurden, nicht erfüllt, einen kombinierten Alarm zu erzeugen, wobei die zwei oder mehr geschätzten neuen Stichproben, die den einen oder die mehreren ähnlichen Tests oder Schwellen nicht erfüllen, identische Fehlermuster einschließen.

7. Verfahren nach Anspruch 5, wobei das eine oder die mehreren Modelle für maschinelles Lernen basierend auf der Passung von zwei oder mehr geschätzten neuen Stichproben, die einen oder mehrere Tests oder Schwellen nicht erfüllen, rekalibriert und aktualisiert werden können, wobei die Trainingsdaten falsch-positive Ergebnisse der Instandhaltungsanalyse einschließen können.

8. Verfahren nach Anspruch 1, ferner umfassend ein Modifizieren eines Betriebs der Industrieanlage (210) basierend auf dem Ausführen der Instandhaltungsanalyse an der bewerteten, geschätzten neuen Stichprobe.

9. System, umfassend:
einen Datenprozessor (225), eine Anzeige (250), eine Steuerung (240) und einen Speicher (245), der nicht flüchtige, computerlesbare Anweisungen speichert, die wenn sie ausgeführt werden, den Datenprozessor (225) veranlassen, Vorgänge, einschließlich des Verfahrens nach einem der vorstehenden Ansprüche, durchzuführen.

10. Nicht flüchtiges, computerlesbares Speichermedium, das Programmanweisungen enthält, die, wenn sie durch mindestens einen Datenprozessor ausgeführt werden, den mindestens einen Datenprozessor veranlassen, Vorgänge, umfassend das Verfahren nach einem der Ansprüche 1 bis 8, durchzuführen.

## Revendications

1. Procédé (100) comprenant :
la réception de données caractérisant des valeurs de données de mesure acquises par un capteur (215) couplé à un actif (210) industriel ;
**caractérisé par** l'identification (110) d'un échantillon de données anormales au sein des données reçues, dans lequel l'identification de l'échantillon de données anormales comporte l'application d'une règle de validation de données aux données reçues, la règle de validation de données étant configurée pour vérifier la qualité des données reçues et pour générer une alerte en réponse au fait que l'échantillon de données anormales ne satisfait pas à la règle de validation de données, dans lequel la règle de validation de données vérifie un type de données des valeurs de données de mesure et un rythme de changement des valeurs de données de mesure, une règle de variance moyenne vérifiant le rythme de changement des valeurs de données de mesure et s'il existe un changement de la valeur moyenne et une variance pour le rythme de changement ;
la suppression de l'échantillon de données anormales,
l'estimation (120), à l'aide d'une interpolation, d'un nouvel échantillon de l'échantillon de données supprimé, dans lequel l'estimation d'un nouvel échantillon de l'échantillon de données supprimé est réalisée sur la base d'une amplification de gradient ;
l'évaluation (130) du nouvel échantillon estimé, dans lequel l'évaluation du nouvel échantillon estimé comporte l'évaluation d'un ajustement du nouvel échantillon estimé vis-à-vis d'un ensemble de données de valeurs de données de mesure connues, l'ajustement étant déterminé sur la base de l'application d'un test F et/ou d'un ou de plusieurs seuils prédéterminés, dans lequel, en réponse à la détermination du fait que l'ajustement du nouvel échantillon estimé échoue aux tests ou n'est pas dans le ou les seuils prédéterminés, la génération d'une alerte comportant au moins une étiquette identifiant l'échec au test ou l'échec au seuil ;
l'exécution (140) d'une analyse de maintenance sur le nouvel échantillon estimé évalué ; et
la fourniture d'un résultat de l'analyse de maintenance, dans lequel l'analyse de maintenance est exécutée sur le nouvel échantillon estimé évalué sur la base de l'au moins une étiquette, dans lequel la fourniture du résultat de l'analyse de maintenance comporte la fourniture de l'alerte et de l'au moins une étiquette dans un afficheur (250).

2. Procédé selon la revendication 1, dans lequel le capteur (215) est apposé sur un actif (210) dans un environnement industriel pétrolier et gazier et les données caractérisent en outre un état de santé de l'actif (210).

3. Procédé selon la revendication 2, dans lequel le capteur (215) est inclus dans un système de surveillance de santé de capteur (200) associé à l'environnement industriel pétrolier et gazier et les données caractérisent en outre un état de santé du capteur (215).

4. Procédé selon la revendication 1, dans lequel la règle de validation de données vérifie en outre au moins l'une parmi une valeur de données vide dans les valeurs de données de mesure, une présence de valeurs de données de mesure répétées, et une absence d'au moins trois valeurs de données de mesure.

5. Procédé selon la revendication 1, dans lequel la génération d'alertes est supprimée sur la base de l'évaluation de l'alerte et de l'ajustement à l'aide d'un ou de plusieurs modèles d'apprentissage automatique entraînés dans un processus d'apprentissage automatique utilisant un historique de valeurs de données de mesure associées à l'actif (210) industriel en tant que de données d'entraînement pour le ou les modèles d'apprentissage automatique.

6. Procédé selon la revendication 5, dans lequel le ou les modèles d'apprentissage automatique sont en outre configurés pour générer une alerte combinée en réponse à la détermination du fait que l'ajustement d'au moins deux nouveaux échantillons estimés échouant à un ou plusieurs tests ou seuils identifiés comme ayant échoué précédemment, dans lequel les au moins deux nouveaux échantillons estimés échouant au ou aux tests ou seuils similaires comportent des profils d'échec identiques.

7. Procédé selon la revendication 5, dans lequel le ou les modèles d'apprentissage automatique peuvent être réétalonnés et mis à jour sur la base de l'ajustement d'au moins deux nouveaux échantillons estimés échouant à un ou plusieurs tests ou seuils, dans lequel les données d'entraînement peuvent comporter des résultats faux positifs de l'analyse de maintenance.

8. Procédé selon la revendication 1, comprenant en outre la modification d'un fonctionnement de l'actif (210) industriel sur la base de l'exécution de l'analyse de maintenance sur le nouvel échantillon estimé évalué.

9. Système comprenant :
un processeur de données (225), un afficheur (250), un dispositif de commande (240) et une mémoire (245) stockant des instructions non transitoires lisibles par ordinateur, qui, lorsqu'elles sont exécutées, amènent le processeur de données (225) à réaliser des opérations comportant le procédé de l'une quelconque des revendications précédentes.

10. Support non transitoire lisible par ordinateur stockant des instructions qui, lorsqu'elles sont exécutées par au moins un processeur de données amènent l'au moins un processeur de données à réaliser des opérations comprenant le procédé de l'une quelconque des revendications 1 à 8.
